# EUROPÄISCHE PATENTSCHRIFT

(11) **EP 0 865 433 B1**
(45) Veröffentlichungstag und Bekanntmachung des Hinweises auf die Patenterteilung: **04.10.2001**
(21) Anmeldenummer: 96938070.8
(22) Anmeldetag: 04.11.1996
(51) Int. Cl.: C07D 235/32

(54) **VERFAHREN ZUR VERMINDERUNG VON NEBENPRODUKTANTEILEN IN CARBENDAZIM DURCH ZUSATZ VON DIAZOTIERUNGSMITTELN**
METHOD OF REDUCING THE AMOUNT OF BY-PRODUCTS IN CARBENDAZIM BY ADDING DIAZOTIZATION AGENTS
PROCEDE POUR REDUIRE LA QUANTITE DE SOUS-PRODUITS DANS LE CARBENDAZIM PAR ADDITION D'AGENTS DE DIAZOTATION

(30) Priorität: 24.11.1995 DE 19543896
(43) Veröffentlichungstag der Anmeldung: 23.09.1998
(73) Patentinhaber: Aventis CropScience GmbH, 65929 Frankfurt am Main (DE)
(72) Erfinder: RESSEL, Hans-Joachim, D-65795 Hattersheim (DE); SCHLEGEL, Günter, D-65835 Liederbach (DE)
(86) Internationale Anmeldenummer: EP9604799
(87) Internationale Veröffentlichungsnummer: WO9719933

(56) Entgegenhaltungen:
- DE-A- 4 402 043
- ULLMANN'S ENCYCLOPEDIA OF CHEMICAL INDUSTRY, Bd. A8, 1987, WEINHEIM, Seiten 505-522, XP002025975 HERTEL,H.: "Diazo Compounds and Diazo Reactions"

## Beschreibung

Die Erfindung betrifft ein Verfahren, das Carbendazim von unzureichender Qualität in ein Produkt hoher Reinheit überführt.

Carbendazim (Methyl-1H-benzimidazol-2-ylcarbamat) (I) ist ein wertvolles Fungizid mit einer großen Wirkungsbreite [Charles R. Worthing, Pesticide Manual (1991) S. 123, The British Crop Protection Council].

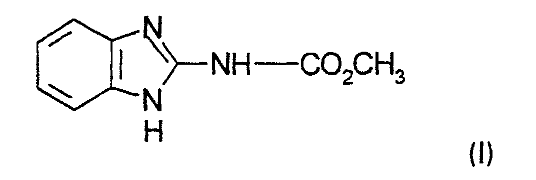

Es kann sowohl als systemisch wirkendes Pflanzenfungizid als auch als technische Fungizid eingesetzt werden. Des weiteren dient es als Rohstoff zur Herstellung des wichtigen Fungizids Benomyl.

Die Herstellung von Carbendazim erfolgt bei allen gängigen Synthesen vorteilhaft durch Umsetzung von 1,2-Diaminobenzol mit Methylcyanocarbamat gemäß DE-A-16 68 557 und DE-A-17 95 849. Bei dieser Reaktion entstehen dimere und polymere Kondensate des 1,2-Diaminobenzols als Nebenprodukte, die zu unerwünschten Verfärbung des Produktes führen und teilweise toxische Eigenschaften besitzen können. Diese Verunreinigungen können auch Eigenschaften besitzen, die zum Teil schon dem eingesetzten 1,2-Diaminobenzol anhaften können.

Von diesen Nebenprodukten seien beispielhaft 2,3-Diaminophenazin (II) und 3-Amino-2-hydroxy-phenazin (III) 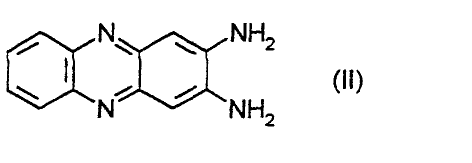 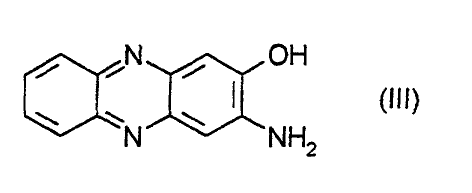 genannt, an deren Restgehalte in auf diese Weise hergestelltem Carbendazim die Reinheit des Produktes gemessen werden kann. Zur Absicherung der Qualität ist es daher von Bedeutung, die färbenden und toxischen Begleitstoffe des Carbendazims zu eliminieren.

Die Verminderung dieser Nebenproduktanteile während der Synthese von Carbendazim durch Zusatz von Reduktionsmitteln (Dithionite und Thiosulfate) ist bereits beschrieben (DE-A-33 23 034). Die Bildung solcher Nebenprodukte kann auch durch konsequentes Arbeiten unter Schutzgas und Verwendung von extrem reinem 1,2-Diaminobenzol bei der Synthese unterdrückt werden.
DE-A-44 02 043 beschreibt die nachträgliche Behandlung von Carbendazim mit einem großem Überschuß an wäßriger Formaldehydlösung.

Nachteilig bei allen bisher bekannten Verfahren ist, daß jeweils nur Ausgangsstoffe bestimmter Reinheit oder Carbendazim-Chargen mit besonderen Eigenschaften verwendet werden können. Hierdurch kann die Bildung der oben genannten Nebenprodukte nicht sicher und breit anwendbar unterdrückt oder deren Gehalt im Umsetzungsprodukt nicht in zufriedenstellendem Maße vermindert werden. Die Prozesse sind zudem durch hohe Kosten und/oder schwierige Entsorgung der anfallenden Mutterlaugen gekennzeichnet.

Es stellt sich daher die Aufgabe, den Gehalt an unerwünschten Komponenten in unreinen Carbendazim-Chargen durch eine einfache, allgemein verwendbare Methode kostengünstig und ohne Belastung der Umwelt wesentlich zu vermindern oder diese Komponenten gänzlich zu beseitigen.

Die Aufgabe wird erfindungsgemäß dadurch gelöst, daß das verunreinigte Carbendazim in saurem, wäßrigem Medium suspendiert, gelöst oder teilweise gelöst mit einer geringen Menge eines Diazotierungsreagenzes versetzt wird. Nach Rückführung zum neutralen pH-Bereich kann auf übliche Weise isoliert werden. Als Diazotierungsmittel kommen bevorzugt salpetrige Säure, deren Salze (Nitrite) und deren wäßrige Lösungen sowie Nitrosylschwefelsäure und deren Lösungen zur Verwendung. Die Säuren sind vorzugsweise Mineralsäuren wie Halogenwasserstoffsäuren (HCl, HBr), Schwefelsäure sowie Phosphorsäure und Salpetersäure. Es können jedoch auch andere Diazotierungsmittel und Säuren verwendet werden.

Die Erfindung betrifft daher ein Verfahren zur Verminderung des Gehalts an unerwünschten Nebenprodukten in unreinem Carbendazim, das dadurch gekennzeichnet ist, daß man das unreine Carbendazim im Sauren mit einem Diazotierungsmittel behandelt und das Carbendazim anschließend in der üblichen Weise isoliert.

Bei dem genannten unreinen Carbendazim handelt es sich vorzugsweise um bei den gängigen industriellen Herstellungsverfahren anfallenden Carbendazim-Chargen, insbesondere das bei der Umsetzung von 1,2-Diaminobenzol mit Methylcyanocarbamat anfallende Produkt.

Beim erfindungsgemäßen Verfahren wird das unreine Carbendazim bei 0 bis 100°C, vorteilhaft bei 0 bis 70°C in verdünnter, wäßriger Säure, vorzugsweise Mineralsäure (0 bis 1,0 Äquivalente pro Mol Wirkstoff) suspendiert bzw. gelöst und mit bis zu 30 g, vorzugsweise 0,5 bis 30 g des Diazotierungsmittels pro kg Carbendazim-Wirkstoff versetzt. Vorzugsweise werden Alkali- oder Erdalkalinitrite oder Nitrosylschwefelsäure in Form einer wäßrigen bzw. schwefelsauren Lösung oder als Feststoff als Diazotierungsreagenz verwendet, wobei bei Einsatz der Nitrosylschwefelsäure ganz oder teilweise auf Zusatz von Mineralsäuren verzichtet werden kann. Nach 0,5 bis 4 Stunden Einwirkzeit wird mit einer Base ein pH-Bereich von 5,5 bis 7 eingestellt. Das reine Carbendazim wird üblicherweise durch Filtrieren/Zentrifugieren in Ausbeuten in der Regel von über 99 % des Einsatzes isoliert. Die wäßrige Mutterlauge kann direkt über die biologische Kläranlage entsorgt werden, wodurch das erfindungsgemäße Verfahren deutliche Vorteile aus ökologischer Sicht bietet.

Die Analyse des so gewonnenen Carbendazims zeigt generell Gehalte der Verunreinigungen der Formel II und III jeweils unter 1 ppm an. Es ist damit für alle Anwendungbereiche verwendbar.

Das Verfahren wird bevorzugt unter einer Inertgasatmosphäre durchgeführt. Die gefundene Lösung des Problems durch die erfindungsgemäße Methode ist überraschend, da Benzimidazol-2-carbamate mit Diazotierungsmitteln selbst reagieren könnten. Diese mögliche Reaktion wird jedoch bei den gewählten Reaktionsbedingungen unterdrückt, es tritt kein Produktverlust auf.

Das erfindungsgemäße Verfahren wird nachfolgend an Beispielen erläutert, ohne daß es auf diese beschränkt wäre.

### Beispiel 1:

40 g Roh-Carbendazim-Pulver (98,5 %ig; II = 49 ppm, III = 2 ppm) werden unter N₂-Atmosphäre mit 100 ml entsalztem Wasser zu einer Suspension verrührt. Bei 60°C werden dann 0,5 ml Nitrosylschwefelsäure, 40 %ig gelöst in H₂SO₄ unter gutem Rühren tropfenweise unter die Flüssigkeitsoberfläche zugesetzt über einen Zeitraum von 30 Minuten. Hierbei hellt sich die anfangs braune Farbe zusehends auf. Man rührt für insgesamt 4 Stunden bei gleicher Temperatur weiter und neutralisiert dann mit verdünnter Natronlauge auf pH 6 bis 7. Das Produkt wird abgesaugt und mit Wasser (50°C) gewaschen. Nach Trocknung erhält man 39,5 g reines Carbendazim ≙99,4 % d. Theorie (HPLC: 99,2 %ig; II < 1 ppm : III < 1 ppm).

### Beispiel 2:

40 g Roh-Carbendazim (98,5 %ig; II = 49 ppm; III = 2 ppm) werden unter N₂-Überlagerung in 90 ml Wasser und 10 ml 2-molarer Salzsäure unter Rühren auf 50°C erwärmt. Bei dieser Temperatur tropft man langsam über 30 Minuten eine Lösung von 0,4 g Natriumnitrit in 10 ml Wasser unter die Flüssigkeitsoberfläche zu und rührt dann für weitere 3 Stunden bei gleicher Temperatur weiter. Anschließend wird der pH-Wert durch Zutropfen von 20 %iger Natronlauge auf 6 eingestellt. Das Produkt wird abfiltriert und mit Wasser (50°C) gewaschen. Nach Trocknung im Vakuum bei 65 bis 70°C werden 39,55 g reines Carbendazim erhalten ≙99,8 % d. Theorie (HPLC: 99,5 %ig; II < 1 ppm; III < 1 ppm).

### Beispiel 3:

40 g Roh-Carbendazim (99,7 %ig; II = 14 ppm; III = 2 ppm) werden unter Stickstoffatmosphäre in 120 ml 2-molarer Salzsäure und 40 ml Wasser durch Erwärmen auf 55°C unter Rühren gelöst. Bei 50°C werden dann 0,4 g kristalline Nitrosylschwefelsäure (E. Merck, Art. Nr. 820911) unter gutem Rühren portionsweise zugesetzt. Man rührt noch für 30 Minuten bei gleicher Temperatur und neutralisiert dann mit 20 %iger Natronlauge. Das ausgefällte Produkt wird über eine Nutsche abgesaugt und dreimal mit wenig Wasser gewaschen.
Nach Trocknung (65°C/100 mbar) erhält man 39,7 g Produkt ≙99,3 % d. Th. (HPLC: 99,8 %ig; II < 1 ppm; III < 1 ppm).

### Vergleichsbeispiel 1:

Man verfährt analog Beispiel 3 (gleiche Einsatz-Charge), jedoch ohne Zusatz von Diazotierungsmittel.
Produkt: 39,7 g = 99,25 % d. Theorie
(HPLC: 99,7 %ig; II = 8 ppm; III = 1,5 ppm).

### Vergleichsbeispiel 2 (entspricht DE-A-44 02 043):

Man verfährt analog Beispiel 1 (gleiche Einsatz-Charge), jedoch Zusatz von 5,0 g Formalin, 37 % wäßrig und 0,05 g Netzmittel (® Präwozell ND 11) statt eines Diazotierungsmittels.
Produkt: 39,6 g ≙99,8 % d. Theorie
(HPLC: 99,3 %ig; II = 3 ppm; III = 1,5 ppm).

## Patentansprüche

1. Verfahren zur Verminderung des Gehalts an unerwünschten Nebenprodukten in unreinem Carbendazim, **dadurch gekennzeichnet, daß** man das unreine Carbendazim im Sauren mit einem Diazotierungsmittel behandelt und das Carbendazim anschließend in der üblichen Weise isoliert.

2. Verfahren gemäß Anspruch 1, **dadurch gekennzeichnet, daß** es sich beim dort erwähnten unreinen Carbendazim um das bei der Umsetzung von 1,2-Diaminobenzol mit Methylcyanocarbamat anfallende Produkt handelt.

3. Verfahren nach Anspruch 1 oder 2, **dadurch gekennzeichnet, daß** bis zu 30 g Diazotierungsmittel pro kg unreinem Carbendazim eingesetzt wird.

4. Verfahren nach einem der Ansprüche 1 bis 3, **dadurch gekennzeichnet, daß** die Behandlung mit dem Diazotierungsmittel bei 0 bis + 100°C vorgenommen wird.

5. Verfahren nach einem der Ansprüche 1 bis 4, **dadurch gekennzeichnet, daß** man die Behandlung mit dem Diazotierungsmittel in mineralsaurem Medium in Lösung oder Suspension durchführt.

6. Verfahren nach einem der Ansprüche 1 bis 5, **dadurch gekennzeichnet, daß** das Diazotierungsmittel ausgewählt wird aus den Salzen der salpetrigen Säure und deren wäßrige Lösungen sowie Nitrosylschwefelsäure und deren Lösungen.

7. Verfahren nach einem der Ansprüche 1 bis 6, **dadurch gekennzeichnet, daß** bei der Behandlung mit dem Diazotierungsmittel die Mineralsäure(n) in wäßriger, verdünnter Form in einer Menge von 0 bis 1,0 Äquivalent Säure pro Mol Wirkstoff verwendet wird/werden.

8. Verfahren nach einem der Ansprüche 1 bis 7, **dadurch gekennzeichnet, daß** man die Behandlung mit dem Diazotierungsmittel unter Inertgas-Atmosphäre durchführt.

## Claims

1. A process for reducing the level of undesirable byproducts in impure carbendazim, which comprises treating the impure carbendazim in an acid medium with a diazotizing agent, and then isolating the carbendazim in a conventional manner.

2. A process as claimed in claim 1, wherein the impure carbendazim mentioned therein is the product obtained from the reaction of 1,2-diaminobenzene with methyl cyanocarbamate.

3. A process as claimed in claim 1 or 2, wherein up to 30 g of diazotizing agent are used per kg of impure carbendazim.

4. A process as claimed in any of claims 1 to 3, wherein the treatment with the diazotizing agent is carried out at 0 to +100°C.

5. A process as claimed in any of claims 1 to 4, wherein the treatment with the diazotizing agent is carried out in solution or suspension in a mineral acid medium.

6. A process as claimed in any of claims 1 to 5, wherein the diazotizing agent is selected from the group consisting of the salts of nitrous acid and their aqueous solutions and nitrosylsulfuric acid and its solutions.

7. A process as claimed in any of claims 1 to 6, wherein, in the treatment with the diazotizing agent, the mineral acid(s) is/are used in aqueous, dilute form in an amount of 0 to 1.0 equivalent of acid per mole of active compound.

8. A process as claimed in any of claims 1 to 7, wherein the treatment with the diazotizing agent is carried out under an atmosphere of inert gas.

## Revendications

1. Procédé de diminution de la teneur en produits intermédiaires non souhaités dans le Carbendazim impur, **caractérisé en ce qu'**on traite le Carbendazim impur dans l'acide avec un agent de diazotation et **en ce qu'**on isole ensuite le Carbendazim de manière usuelle.

2. Procédé selon la revendication 1, **caractérisé en ce que** pour le Carbendazim impur revendiqué, il s'agit du produit résultant de la réaction de 1,2-diaminobenzène avec le méthylcyanocarbamate.

3. Procédé selon la revendication 1 ou 2, **caractérisé en ce qu'**on met en oeuvre jusqu'à 30 g d'agent de diazotation par kg de Carbendazim impur.

4. Procédé selon l'une des revendications 1 à 3, **caractérisé en ce que** le traitement avec l'agent de diazotation est réalisé entre 0 et + 100°C.

5. Procédé selon l'une des revendications 1 à 4, **caractérisé en ce qu'**on effectue le traitement avec l'agent de diazotation en solution ou en suspension dans un milieu acide minéral.

6. Procédé selon l'une des revendications 1 à 5, **caractérisé en ce que** l'agent de diazotation est choisi parmi les sels de l'acide nitreux et de leurs solutions aqueuses ainsi que de l'acide nitrosylsulfurique et de leurs solutions.

7. Procédé selon l'une des revendications 1 à 6, **caractérisé en ce que** dans le traitement avec l'agent de diazotation le ou les acides minéraux sont utilisés sous forme aqueuse, diluée en une quantité de 0 à 1,0 équivalent d'acide par mole de substance active.

8. Procédé selon l'une des revendications 1 à 7, **caractérisé en ce qu'**on réalise le traitement avec l'agent de diazotation sous une atmosphère de gaz inerte.
